# EUROPEAN PATENT SPECIFICATION

(11) **EP 1 908 818 B1**
(45) Date of publication and mention of the grant of the patent: **15.10.2014**
(21) Application number: 06781332.9
(22) Date of filing: 20.07.2006
(51) Int. Cl.: C12N 1/14, A23L 1/105, A23L 1/238, C12N 9/30, C12N 9/42, C12N 9/58, C12G 3/00

(54) **PROCESS FOR PRODUCTION OF LIQUID KOJI**
VERFAHREN ZUR HERSTELLUNG VON FLÜSSIGEM KOJI
PROCEDE DE PRODUCTION DE KOJI LIQUIDE

(30) Priority: 22.07.2005 JP 2005212290; 04.10.2005 JP 2005290651
(43) Date of publication of application: 09.04.2008
(73) Proprietor: Asahi Breweries, Ltd., Sumida-ku Tokyo 130-8602 (JP)
(72) Inventor: SUGIMOTO, Toshikazu Asahi Breweries, Ltd, Moriya-shi, Ibaraki 30201 (JP); SHOJI, Hiroshi, Asahi Breweries, Ltd., Moriya-shi, Ibaraki 3020106 (JP)
(74) Representative: Müller, Christian Stefan Gerd
(86) International application number: PCT/JP2006/314372
(87) International publication number: WO 2007/010979

(56) References cited:
- JP-A- 2003 047 455
- JP-A- 2005 318 886
- SHOJI ET AL: "Simultaneous production of glucoamylase and acid-stable alpha-amylase using novel submerged culture of Aspergillus kawachii NBRC4308" JOURNAL OF BIOSCIENCE AND BIOENGINEERING, ELSEVIER, AMSTERDAM, NL, vol. 103, no. 2, 1 February 2007 (2007-02-01), pages 203-205, XP005939375 ISSN: 1389-1723
- BLANDINO A. ET AL.: 'Utilization of whole wheat flour for the production of extracellular pectinases by some fungal strains' PROCESS BIOCHEMISTRY vol. 37, no. 5, 2001, pages 497 - 503, XP003007639
- WAKO H. ET AL.: 'Ekitai Kojiho ni yoru Komezu Moromi no Seizo' HIROSHIMA-KEN SHOKUHIN KOGYO SHIKENJO KENKYU HOKOKU vol. 15, 1980, pages 13 - 19, XP003007640
- SREEKANTIAH K.R. ET AL: 'Effekt of Cultural and Nutritional Variations on Certain Exo-Enzymes Secreted by Fungi' CHEM. MIKROBIOL. TECHNOL. LEBENSM. vol. 2, no. 2, 1973, pages 42 - 48, XP003007641
- SHIBATA S. AND NAKAE T.: 'Revised and enlarged Komugiko Seihin no Chishiki', 2000, KABUSHIKI KAISHA SAIWAI SHOBO pages 72 - 73, XP003007642
- SOTOIKE R.: 'Sake no Jiten' KABUSHIKI KAISHA TOKYODO SHUPPAN 1980, pages 79 - 81, XP003007643

## Description

### Technical Field

The present invention relates to a method of producing liquid koji, in specific, a method of producing liquid koji having enhanced enzymatic activity.

### Background Art

As for koji used in production of alcoholic beverages, there are solid koji, which is cultured such that spores of filamentous fungi are inoculated to raw material which has been treated with cooking and the like, and liquid koji, which is cultured such that liquid medium is prepared by adding raw material and other nutrient sources to water, and then spores of koji molds or pre-cultured mycelia of koji molds and the like are inoculate thereto.

In the conventional production of fermented foods and drinks such as alcoholic beverages including, for example, sake, shochu, soy sauce, fermented soybean paste, sweet sake and the like, what is called solid koji which is prepared with the solid culture method has been widely used. The solid culture method is the culture method in which spores of koji molds such as *Aspergillus kawachii, Aspergillus awamori, Aspergillus niger, Aspergillus oryzae, Aspergillus sojae* and the like are dispersed on solid raw material such as steam-cooked cereals to allow koji molds to grow on the solid surface.

For instance, for the production of shochu, the solid koji such as *Aspergillus kawachii* and *Aspergillus awamori* have been widely used. However, as the solid culture method is a culture system in which raw materials and koj i molds disperse unevenly, it is difficult to make even the factors such as temperature, water content, and various nutrient compositions, and the solid culture method is very complicated in culture control. In addition, the production of koji is often conducted under open conditions, and cares are required in terms of quality control so as to prevent contamination with other bacteria. Therefore, the solid culture method is unsuitable for large-scale production.

In contrast, the liquid culture method is easy to culture control and quality control, so it is suitable for efficient production. However, due to the reason that, for example, enzymatic activity is insufficient for brewing shochu, the culture product obtained by liquid culturing koji molds is rarely used as shochu koji. The culture product obtained by the liquid culture method may be a culture product itself obtained by the liquid culture method (hereinafter, also referred to as "liquid koji"), as well as a culture liquid, cells, and a concentrate thereof, or a dried product thereof.

In addition to the above-mentioned reasons, a major reason of the culture product obtained with the liquid culture method not being used for producing fermented foods and drinks such as shochu is that the behavior of koji molds to produce enzymes such as amylase and cellulase in the liquid culture is known to be much different from that in the solid culture, and productivity thereof is also known to be poor overall (see Non-Patent Documents 1 and 2).

In production of the alcoholic beverages such as shochu, alcohol is usually generated by simultaneous saccharification and fermentation. Therefore, saccharolytic enzymes fromkoji molds, which affect supplying glucose to the koji molds, particularly glucoamylase and acid-stable α-amylase are key enzymes in the alcoholic fermentation. However, it is known that activity of glucoamylase is remarkably low in the culture product obtained with the liquid culture method and production behavior thereof is also much different from that in the solid culture (see Non-Patent Documents 3 to 6).

As a method of improving glucoamylase activity of koji molds, there are reported the method of culturing koji molds while giving stresses on the growth of mycelia (see Patent Document 1) and the method of adding roasted cereals to koji mold culture fluid (see Patent Document 2). The method disclosed in Patent Document 1 conducts culture on porous membrane or in inclusive immobilization agent having air gaps to allow expression of the novel gene glaB that encodes glucoamylase, to thereby enhance enzymatic activity.

Accordingly, the method requires strict control or specific culture devices, and thus it is not practical. The method disclosed in Patent Document 2 cultures koji molds in liquid medium using roasted cereals as, at least, a portion of the raw material, which requires an additional production step of roasting cereals.

The inventors of the present invention provided an invention related to a method of culturing koji molds using liquid medium containing the saccharides which the koj i molds hardly decompose (see Patent Document 3). By liquid culturing koji molds with the invention, a koji mold culture product having high activity of glycolytic enzymes such as glucoamylase, which can be used for producing fermented foods and drinks such as sake, can be obtained conveniently and inexpensively.

On the other hand, recently, the molecular biological analysis on acid-stable α-amylase has been conducted to the details (see Non-Patent Document 7). The analysis has reported as follows: A white koji mold has two different amylase genes which are respectively responsible for two different characteristics, that is, acid-unstable α-amylase and acid-stable α-amylase. The expression behaviors of the respective genes are much different fromeach other. In liquid culturing, the acid-unstable α-amylase is sufficiently produced, while the acid-stable α-amylase, a key enzyme for brewing shochu is hardly produced.

For producing shochu, brewing is conducted under low-pH environments for preventing the shochumash fromputrefaction. The acid-unstable amylase contributes very little to glycolysis in shochubrewing because it is deactivated promptly under low-pH conditions. Therefore, it is indispensable for producing shochu that the acid-stable α-amylase is produced with high yield, which is thought to contribute to the glycolysis in shochu brewing, by liquid culturing koj i molds.

The production behavior of acid stable α-amylase in liquid culturing koji molds has been investigated in detail and reported. However, the method uses synthetic medium containing peptone and citrate buffer solution, and requires an culture time of 100 hours or more, so it would be difficult to apply to actual shochu brewing (see Non-Patent Documents 8 to 10).

As described above, it is thought in general that the acid-stable α-amylase is the enzyme which cannot be produced basically by liquid culturing, and thus, liquid koji having high acid-stable α-amylase activity has not been developed.

A method for producing liquid malted rice having high sugar alcohol concentration has been disclosed (see Patent Document 4). A submerged mold culture fluid for rice vinegar mash has been prepared (see Non-Patent Document 11). The effect of cultural and nutritional variations on certain exo-enzymes secreted by fungi has been investigated (see Non-Patent Document 12).
Patent Document 1: JP 11-225746 A
Patent Document 2: JP 2001-321154 A
Patent Document 3: JP 2003-265165 A
Patent Document 4: JP 2003-047455 A
Non-Patent Document 1: Iwashita K. et al: Biosci.
Non-Patent Document 1: Iwashita K. et al: Biosci. Biotechnol. Bioche., 62, 1938-1946(1998)
Non-Patent Document 2: Yuichi Yamane et al.: Journal of the Brewing Society of Japan, 99, 84-92(2004)
Non-Patent Document 3: Hata Y. et al . : J. Ferment. Bioeng. , 84, 532-537 (1997)
Non-Patent Document 4: HataY. et al. : Gene., 207, 127-134 (1998)
Non-Patent Document 5: Ishida H. et al.: J. Ferment. Bioeng., 86, 301-307 (1998)
Non-Patent Document 6: Ishida H. et al.: Curr. Genet., 37, 373-379 (2000)
Non-Patent Document 7: Nagamine K. et al.: Biosci. Biotechnol. Biochem, 67, 2194-2202 (2003)
Non-Patent Document 8 : Sudo S . et al.: J. Ferment . Bioeng. , 76, 105-110 (1993)
Non-Patent Document 9 : Sudo S . et al.: J. Ferment. Bioeng., 77, 483-489 (1994)
Non-Patent Document 10: Shigetoshi Sudo et al. : Journal of the Brewing Society of Japan, 89, 768-774 (1994)
Non-Patent Document 11: Wadaka, H., et al.: Hiroshima-ken Shokuhin Kogyo Shikenjo Kenkyu Hokoku, 15, 13-19 (1980)
Non-Patent Document 12: Sreekantiah, K.R., et al.: Chem. Mikrobiol. Technol Lebensm., 2, 42-48 (1973)

### Disclosure of Invention

### Problem that the invention is to solve

The inventors of the present invention have found out that liquid koji sufficiently having activity of enzymes such as glucoamylase, acid-stable α-amylase and the like, which are necessary for producing shochu and the like, can be produced by culturing koj i molds in liquid medium containing the cereals of which surface is entirely or partly covered with husks, as culture raw material, and have already filed for patent application (see, the specifications of JP Patent Application No. 2004-350661 and JP Patent Application No. 2004-352320).

However, the production behavior of the enzymes except glucoamylase and acid-stable α-amylase in these methods has remained unknown.

An object of the present invention is to develop a method of enhancing enzymatic activity of amylolytic enzymes such as glucoamylase and acid-stable α-amylase, and the other enzymes in liquid koji. To be specific, an object of the present invention is to provide a method of producing liquid koj i having high enzymatic activity by optimizing composition of the liquid medium.

### Means for solving the problem

In view of achieving higher production of enzymes in liquid koji, the inventors of the present invention have made extensive studies on combination effects of the above-mentioned culture raw materials and various nutrient sources, and have found out that productivity of the glucoamylase that is an amylolytic enzyme, the cellulase that is a cellulolytic enzyme, and the acidic carboxypeptidase that is a proteolytic enzyme can be improved by incorporating a specific nitrogen source in the liquid medium and additionally allowing the nitrogen source to coexist with at least one of a sulfate and a phosphate, and thus have completed the invention.

That is, according to a first aspect of the present invention, there is provided a method of producing liquid koj i having enhanced activity of enzymes, the method comprising culturing white koji molds and/or black koj i molds in a liquid medium which contains (i) a cereal the surface of which is entirely covered with husks and (ii) at least one nitrogen source selected from the group consisting of potassium nitrate, sodium nitrate, yeast cells, treated products of yeast cells, cereal husks and cereal bran.

According to a second aspect of the present invention, there is provided the method of producing liquid koji having enhanced activity of enzymes according to the first aspect, wherein the enzymes comprise glucoamylase and acid-stable α-amylase.

According to a third aspect of the present invention, there is provided the method of producing liquid koji having enhanced activity of enzymes according to the first aspect, wherein the liquid medium contains a cereal the surface of which is entirely covered with husks; at least one nitrogen source selected from the group consisting of potassium nitrate and sodium nitrate; at least one phosphate salt selected from the group consisting of potassium dihydrogen phosphate and ammonium phosphate; and at least one sulfate salt selected from the group consisting of magnesium sulfate heptahydrate, iron sulfate heptahydrate and ammonium sulfate.

According to a fourth aspect of the present invention, there is provided the method of producing liquid koji having enhanced activity of enzymes according to the third aspect, wherein the liquid medium contains the at least one nitrogen source in a concentration of 0.1 to 2.0% (w/vol).

According to a fifth aspect of the present invention, there is provided the method of producing liquid koji having enhanced activity of enzymes according to the third aspect, wherein the liquid medium contains the at least one phosphate salt in a concentration of 0.05 to 1.0% (w/vol).

According to a sixth aspect of the present invention, there is provided the method of producing liquid koji having enhanced activity of enzymes according to the third aspect, wherein the liquid medium contains the at least one sulfate salt in a concentration of 0.01 to 0.5% (w/vol).

According to a seventh aspect of the present invention, there is provided the method of producing liquid koji having enhanced activity of enzymes according to the first aspect, wherein the cereal comprises at least one selected from the group consisting of crude rice, rice with all chaffs, rice with part of chaffs and barley having a polishing ratio of not less than 93%.

According to an eighth aspect of the present invention, there is provided the method of producing liquid koji having enhanced activity of enzymes according to the first aspect, wherein the white koji molds comprise *Aspergillus kawachii.*

According to a ninth aspect of the present invention, there is provided the method of producing liquid koji having enhanced activity of enzymes according to the first aspect, wherein the black koji molds comprise at least one selected from the group consisting of *Aspergillus awamori* and *Aspergillus niger.*

According to a tenth aspect of the present invention, there is provided the method of producing liquid koji having enhanced activity of enzymes according to the first aspect, wherein the cereal comprises barley.

According to an eleventh aspect of the present invention, there is provided a method of producing at least one enzyme selected from the group consisting of glucoamylase and acid-stable α-amylase, comprising producing the at least one enzyme by culturing white koji molds and/or black koji molds in a liquid medium which contains (i) a cereal the surface of which is entirely covered with husks and (ii) at least one nitrogen source selected from the group consisting of potassium nitrate, sodiumnitrate, yeast cells, treatedproductsofyeast cells, cereal husks and cereal bran.

According to a twelfth aspect of the present invention, there is provided a method of producing at least one enzyme selected from the group consisting of glucoamylase, acid-stable α-amylase, cellulase and acidic carboxypeptidase comprising producing the at least one enzyme by culturing white koji molds and/or black koji molds in a liquid medium which contains a cereal the surface of which is entirely covered with husks; at least one nitrogen source selected from the group consisting of potassium nitrate and sodium nitrate; at least one phosphate salt selected from the group consisting of potassium dihydrogen phosphate and ammonium phosphate; and at least one sulfate salt selected from the group consisting of magnesium sulfate heptahydrate, iron sulfate heptahydrate and ammonium sulfate.

According to a thirteenth aspect of the present invention, there is provided the method of producing enzymes according to the twelfth aspect, wherein the liquid medium contains the at least one nitrogen source in a concentration of 0.1 to 2.0% (w/vol).

According to a fourteenth aspect of the present invention, there is provided the method of producing enzymes according to the twelfth aspect, wherein the liquid medium contains the at least one phosphate salt in a concentration of 0. 05 to 1.0% (w/vol).

According to a fifteenth aspect of the present invention, there is provided the method of producing enzymes according to the twelfth aspect, wherein the liquid medium contains the at least one sulfate salt in a concentration of 0.01 to 0.5% (w/vol).

According to a sixteenth aspect of the present invention, there is provided the method of producing enzymes according to the eleventh or twelfth aspect, wherein the cereal comprises at least one selected from the group consisting of crude rice, rice with all chaffs, rice with part of chaffs and barley having a polishing ratio of not less than 93%.

According to a seventeenth aspect of the present invention, there is provided the method of producing enzymes according to the eleventh or twelfth aspect, wherein the white koji molds comprise *Aspergillus kawachii.*

According to an eighteenth aspect of the present invention, there is provided the method of producing enzymes according to the eleventh or twelfth aspect, wherein the black koji molds comprise at least one selected from the group consisting of *Aspergillus awamori* and *Aspergillus niger.*

According to a nineteenth aspect of the present invention, there is provided the method of producing enzymes according to the eleventh or twelfth aspect, wherein the cereal comprises barley.

### Effect of the invention

According to the present invention, a specific organic substance and/or an inorganic substance as nitrogen source are added to liquid medium containing the cereal of which surface is entirely or partly covered with husks as culture raw material, a sulfate salt and a phosphate salt are further added thereto, and koji molds are cultured in the liquid medium, not only to remarkably improve productivity of amylolytic enzymes in liquid koji, but also to produce liquid koji containing cellulolytic enzymes and proteolytic enzymes with high yield. In addition, it is thought that productivity of the enzymes produced by the koji molds is generally improved even other than the above-mentioned enzymes.

When fermented foods and drinks such as shochu are produced by using the liquid koji produced according to the present invention, good fermentation is performed owing to decrease in mash viscosity because of high activity of cellulolytic enzymes, whereby increase in alcohol yield can be expected. In addition, production of amino acids is increased owing to high activity of proteolytic enzymes, whereby fermented foods and drinks having gorgeous flavor can be produced.

Further, the liquid culturing can control strictly as compared to the solid culturing, so liquid koj i having quality stability can be produced at low cost.

Besides, the cereals used in the present invention are unpolished or polished to the extent that at least husks are remained on the surface. Thus, improvement in raw material availability and in yield can be expected.

### Brief description of the drawings

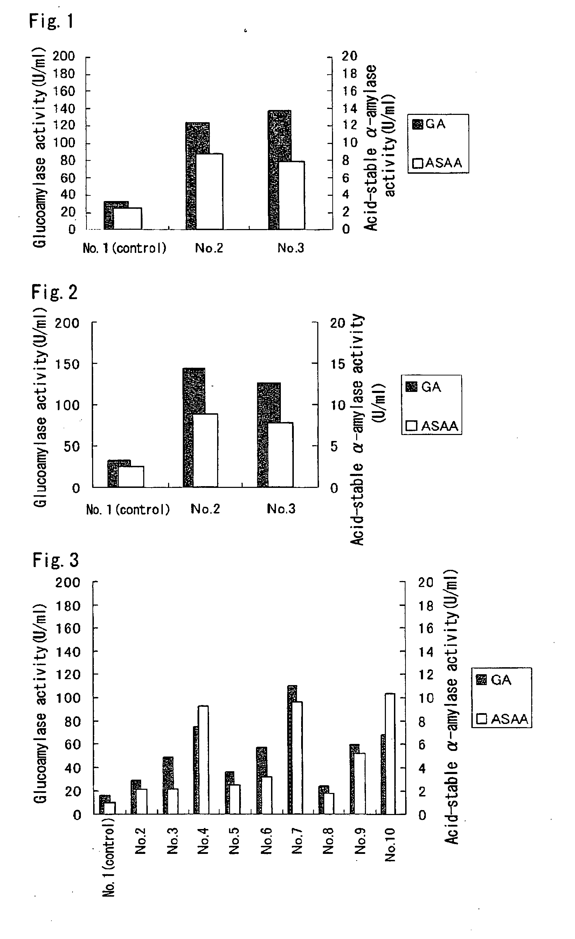

FIG. 1 shows glucoamylase and acid-stable α-amylase activities of culture products each obtained by culturing with the liquid medium using potassium nitrate as nitrogen source. Black bars each represent glucoamylase activity (U/ml), and white bars each represent acid-stable α-amylase activity (U/ml).
FIG. 2 shows glucoamylase and acid-stable α-amylase activities of culture products each obtained by culturing with the liquid medium using an inorganic nitrogen substance and an inorganic salt. Black bars each represent glucoamylase activity (U/ml), and white bars each represent acid-stable α-amylase activity (U/ml).
FIG. 3 shows glucoamylase and acid-stable α-amylase activities of culture products each obtained by culturing with the liquid medium using yeast cells or yeast autolysate as nitrogen source. Black bars each represent glucoamylase activity (U/ml), and white bars each represent acid-stable α-amylase activity (U/ml).

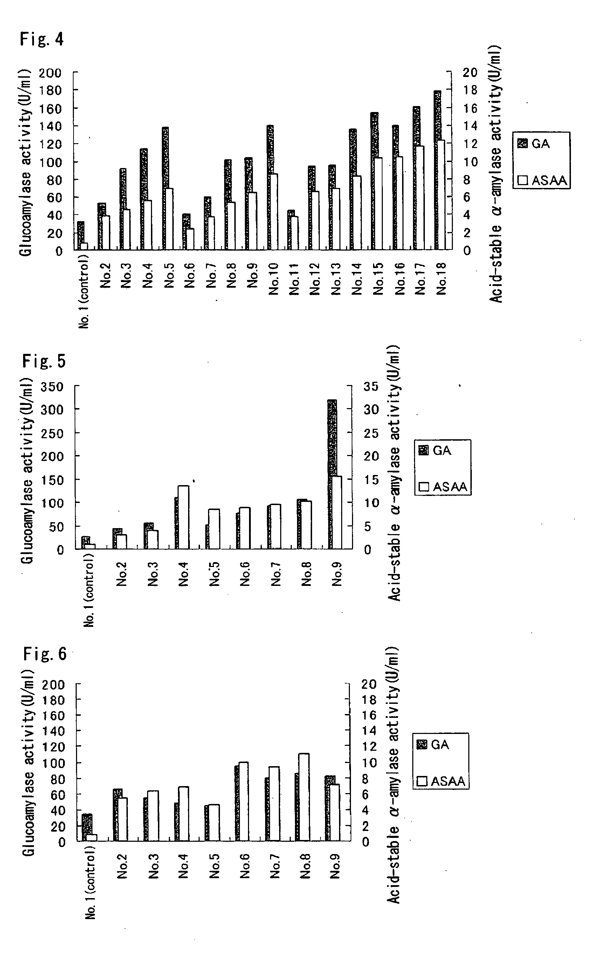

FIG. 4 shows glucoamylase and acid-stable α-amylase activities of culture products each obtained by culturing with the liquid medium using a combination of an inorganic nitrogen substance, an inorganic salt, and yeast cells. Black bars each represent glucoamylase activity (U/ml), and white bars each represent acid-stable α-amylase activity (U/ml).
FIG. 5 shows glucoamylase and acid-stable α-amylase activities of culture products each obtained by culturing with the liquid medium using a combination of barley bran, yeast cells and an inorganic nitrogen substance as nitrogen source. Black bars each represent glucoamylase activity (U/ml), and white bars each represent acid-stable α-amylase activity (U/ml).
FIG. 6 shows glucoamylase and acid-stable α-amylase activities of culture products each obtained by culturing with the liquid medium using a combination of barley husks and yeast cells as nitrogen source. Black bars each represent glucoamylase activity (U/ml), and white bars each represent acid-stable α-amylase activity (U/ml).

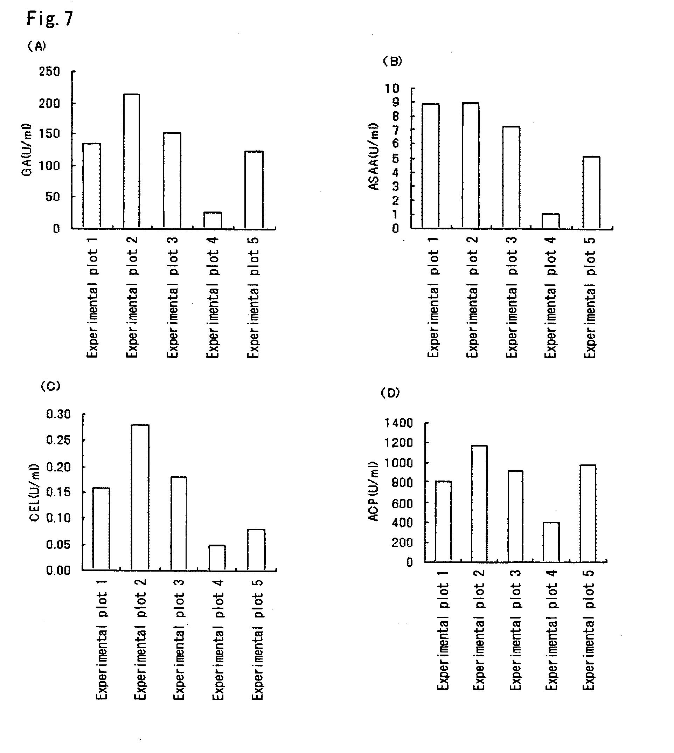

FIG. 7 shows activities of various enzymes of koji mold culture products each obtained by culturing with the liquid medium using a sulfate salt, a nitrate salt and a phosphate salt. From (A) to (D) show respectively activities (U/ml) of glucoamylase (GA), acid-stable α-amylase (ASAA), cellulase (CEL) and acid carboxypeptidase (ACP).

### Best embodiments for carrying out the invention

Hereinafter, the present invention will be described in detail.

The method of producing liquid koji according to the present invention comprises the step of culturing koji molds in the liquid medium prepared by adding thereto raw materials such as cereals and nitrogen sources to produce liquid koji with enhanced enzymatic activity.

More specifically, in the present invention, koji molds are cultured with the liquid medium that contains the cereals the surface of which is entirely covered with husks, and thus, it takes time for saccharifying starches in the cereals, releasing rate of the saccharides into the culture system is suppressed, whereby enzymatic activity of the liquid koji is enhanced. Further, various enzymes are highly produced by the koji molds because the liquid medium contains specific nutrient sources.

Here, examples of the enzyme to be produced by the koji molds comprise, but not necessarily limited to, an amylolytic enzyme such as glucoamylase and α-amylase, a cellulolytic enzyme such as cellulase and β-glucosidase, and a proteolytic enzymes such as acid carboxypeptidase and acid protease.

In the present invention, examples of the cereal to be used as culture raw material comprise barley, rice, wheat, buckwheat, barnyard millet, foxtail millet, millet, kaoliang, corn and the like. Each of the culture raw materials needs to have a form such that its surface is entirely covered with husks. There can be used an unpolished stuff or that having equal to or more of the polishing ratio at which it has been polished so that husks are at least remained on the surface of kernels, and crude rice, crude barley and the like also can be used. In the case of rice, crude rice, rice with all chaffs and rice with part of the chaffs may be used.

For instance, when the cereal is barley, there can be used unpolished stuff having a polishing ratio of 100%, or provided that the polishing ratio of the unpolished stuff is defined as of 100%, the stuff having the polishing ratio determined by subtracting the husk ratio of barley (generally 7 to 8%) from the polishing ratio of the unpolished stuff, i.e., that having polishing ratio of not less than 93%.

Here, the term "polishing ratio" refers to the remained percentage after polishing the cereals. For instance, the term "polishing ratio of 90%" means that 10% of the husks or the like on the surface layer portion of cereals is shaved away. In the present invention, furthermore, the term "crude barley" comprises those from unpolished barley to polished barley having husks remained on the kernels' surfaces, that is, the stuff having polishing ratio of 90% or more. In addition, the term "husks" refers to the outside part that covers the surface of a cereal particle.

Any one of the above-mentioned culture raw materials is used alone, or two or more of them are used in combination for preparing the following liquidmedium. That is, the cereal as culture raw material is mixed with water in combination with the nitrogen source described herein below to prepare the liquid medium. A blending ratio of the cereal is adjusted to the extent that enzymes such as an amylolytic enzyme, a cellulolytic enzyme, and a proteolytic enzyme are selectively generated and accumulated in the culture product of koj i molds.

For instance, when barley is used as culture rawmaterial, liquid medium is prepared by adding 1 to 20% (w/vol) of crude barley to water. Whenunpolishedbarleyisusedas crude barley, more preferably, liquid medium is prepared with the addition of 8 to 10% (w/vol). When 95%-polished barley as crude barley is used as raw material, more preferably, liquid medium is prepared with the addition of 1 to 4% (w/vol).

In addition, when crude rice from which chaffs are removed is used as culture raw material, liquid medium is prepared by adding 1 to 20% (w/vol) of the crude rice to water, preferably 5 to 13% (w/vol), more preferably 8 to 10% (w/vol).

When the other cereals are used, liquid medium is prepared in the same manner by adding 1 to 20% (w/vol) of the cereals to water.

In this manner, the most suitable blending amount varies depending on polishing degrees of raw material to be used, koji strains to be used, kinds of the raw material and the like, so may be appropriately selected with considering these.

When the amount of the culture raw material used exceeds the upper limit value, viscosity of the culture medium rises, and supply of oxygen or air required for aerobically culturing koji molds becomes insufficient to allow culture progress poor, and it is not preferred. On the other hand, when the amount of the raw material used does not satisfy the lower limit value, the desirable enzymes cannot be produced with high yield.

Starches included in the culture raw material may be gelatinized before culturing. A method of gelatinizing starches is not particularly limited, and may be conducted according to any of the conventional methods comprising the steaming method and the roasting method. In the step of sterilizing liquid medium as described later, if the starches are heated to the gelation temperature or higher by sterilization at high temperatures and high pressures, the gelatinization of starches is simultaneously conducted by such a treatment.

In the liquid medium, an organic substance, an inorganic substance and the like are included as nitrogen source in addition to the above-mentioned culture raw material. The nitrogen source is selected from the group consisting of potassium nitrate, sodium nitrate, yeast cells, treated products of yeast cells (such as decomposed yeast cells, yeast extracts and the like), cereal husks, and cereal bran. Potassium nitrate is particularly preferred.

Any one of the nitrogen sources may be used alone, or two or more of the organic substances and/or the inorganic substances may be used in combination.

The addition amount of the nitrogen source is not particularly limited as long as growth of the koji molds is promoted, however, 0.1 to 2% (w/vol), preferably 0.5 to 1.0% (w/vol) as organic substance, and the addition amount of the nitrate salt as inorganic substance is 0.05 to 2.0% (w/vol), preferably 0.1 to 2.0% (w/vol), more preferably 0.2 to 1.5% (w/vol).

Addition of the nitrogen source in an amount exceeding the upper limit value is not preferable because growth of the koj i molds is inhibited. On the other hand, an addition amount of the nitrogen source lower than the lower limit value is also not preferable because enzyme production is not promoted.

Examples of the yeast to be used as one kind of the nitrogen source in the present invention comprise beer yeast, wine yeast, whisky yeast, shochu yeast, sake yeast and bread yeast which are used in brewing or food production processes, and the yeast cells of genera *Saccharomyces, Candida, Torulopsis, Hanseniaspora, Hansenula, Debaryomyces, Saccharomycopsis, Saccharomycodes, Pichia, Pachysolen* and the like.

The cells of the yeast themselves can be used as nitrogen source, and also can be used in the form of decomposed yeast cells or yeast extract. The decomposed yeast cells or the yeast extract can be obtained by subjecting yeast cells to treatments such as the autolysis method (method of solubilizing cells utilizing proteolytic enzymes which originally exists in the yeast cells), the enzymolysis method (method of solubilizing with addition of an enzyme preparation or the like derived from microorganisms or plants), the hot water extraction method (method of solubilizing by immersing yeast cells in hot water for a certain period of time), the acid or alkaline decomposition method (method of solubilizing with addition of various acids or alkalis), the physical crushing method (method of crushing with the ultrasonication treatment and the high-pressure homogenization method, or by mixing with solids such as a glass beads and stirring), and the freeze-thaw method (method of crushing by freezing and thawing at least once).

In addition, cereal bran such as rice bran, which is a by-product obtained from polishing cereals, can also be used as nitrogen source. A seed of cereal is composed of a testa portion, an embryo portion, an endosperm portion and chaff protecting these. The bran is a portion composed of the embryo and the testa portion.

Further, in the present invention, cereal husks, that is a testa portion of cereals, can be used as nitrogen source, and cereal husks of the same kind of the cereal used as culture raw material is generally used. Those cereal bran and cereal husks can be used in combination with the other nitrogen sources.

In the liquid medium to be used in the present invention, a sulfate salt and a phosphate salt may be included in addition to the culture raw material and the nitrogen sources as described above. By using those inorganic salts in combination, it becomes possible to enhance enzymatic activity of an amylolytic enzyme, a cellulolytic enzyme, a proteolytic enzyme and the like.

Examples of the sulfate salt comprise magnesium sulfate heptahydrate, iron sulfate heptahydrate and ammonium sulfate, and magnesium sulfate heptahydrate is specifically preferable. Examples of the phosphate salt comprise potassium dihydrogen phosphate and ammonium phosphate, and potassium dihydrogen phosphate is specifically preferable.

Any one of those inorganic salts can be used alone, or two or more of them can be used in combination.

In addition, the concentration of the inorganic salts in liquid medium is adjusted to the extent that the enzymes such as an amylolytic enzyme, a cellulolytic enzyme, and a proteolytic enzyme are selectively generated and accumulated in the culture product of koji molds. For instance, the concentration of the sulfate salt is 0.01 to 0.5%, preferably 0.02 to 0.1%, and the concentration of the phosphate salt is 0.05 to 1.0%, preferably 0.1 to 0.5%, provided that, every value is in w/vol.

The above-mentioned inorganic salts may be used alone, or two or more of them may be used in combination.

To the liquid medium, an organic substance and an inorganic salt may optionally be added other than the above-mentioned nitrogen source and inorganic salt as nutrient source. The additives are not particularly limited as long as they are the substances generally used for culturing koji molds. Examples of the organic substance comprise wheat bran, corn steep liquor, a soybean cake and defatted soybean. Examples of the inorganic salt comprise water-soluble compounds such as an ammonium salt, a potassium salt, a calcium salt, a magnesium salt and the like. Two or more organic substances and/or inorganic salts may be used at the same time.

The addition amount thereof is not particularly limited as far as growth of the koj i molds is facilitated. The addition amount of the organic substance is preferably about 0.1 to 5% (w/vol) and the addition amount of the inorganic salt is preferably about 0.1 to 1% (w/vol).

Addition of the nutrient source in an amount exceeding the upper limit value is not preferable because growth of the koj i molds is inhibited. On the other hand, an addition amount of the nutrient source lower than the lower limit value is also not preferable because enzyme production is not promoted.

The liquid medium for koji molds obtained by mixing the above-mentioned culture raw material and the nitrogen source with water may optionally be subjected to a sterilization treatment, and the treatingmethod is not particularly limited. An example of the method comprises the high-temperature and high-pressure sterilization method, and in this case, sterilization may be conducted at 121°C for 15 minutes.

The sterilized liquid medium is cooled down to a culture temperature and then white koji molds and/or black koji molds are inoculated to the liquid medium.

Examples of the koji molds to be used in the present invention preferably comprise koj i molds capable of producing amylolytic enzymes such as glucoamylase, acid-stable α-amylase and α-amylase, cellulolytic enzymes such as cellulase and β-glucosidase, and proteolytic enzymes such as acid carboxypeptidase and acid protease. Specific examples of the koj i molds comprise white koj i molds such as *Aspergillus kawachii*, and black koji molds such as *Aspergillus awamori* and *Aspergillus niger.*

The form of the koj i molds to be inoculated to the medium is arbitrary, and any one of the spores and the mycelia of the koji molds can be used.

Those koj i molds may be used for the single strain culture or for the mixed culture with two or more homologous or heterologous strains. It is allowed to use either form of the spores or the mycelia obtained in preculture. However, the mycelia is preferably used because shorter period of time is required for the logarithmic growth phase.

The amount of the koji molds inoculated into the liquid medium is not particularly limited but the number of the spores may be in the range of about 1 x 10⁴ to 1 x 10⁶ per ml of the liquid medium. For the mycelia, about 0.1 to 10% of the preculture liquid is preferably inoculated.

The culture temperature of the koj i molds is preferably 25 to 45°C, more preferably 30 to 40°C but not particularly limited as far as the growth is not harmfully affected. If the culture temperature is low, it tends to be contaminated with infectious microbes as growth of the koji molds becomes slow. Thus, the culture time is appropriately in the range of 24 to 72 hours.

The culture apparatus may be any of those capable of carrying out liquid culture. The koji molds have to be cultured aerobically. Thus, the culture should be conducted under aerobic conditions in which oxygen or air can be supplied into the medium. In addition, it is preferable to stir the medium so that the raw materials, oxygen, and the koji molds can be uniformly distributed in the apparatus during culture. The stirring conditions and the amount of aeration may be conducted under any conditions so far as an aerobic culture environment is maintained and thus may be appropriately selected depending on the culture apparatus, the viscosity of medium and the like.

By culturing with the above culture method, the enzymes such as an amylolytic enzyme, a cellulolytic enzyme, and a proteolytic enzyme and the like can be highly produced. As a result, liquid koji having the enzymatic activity to be used for brewing shochu is obtained.

The liquid koji produced according to the present invention comprises the culture fluid obtained from the culture product by centrifugal separation and the like, the concentrate thereof, the dried product thereof and the like, as well as the culture product itself.

As described above, according to the above-described culture method, the enzymes such as an amylolytic enzyme, a cellulolytic enzyme, and a proteolytic enzyme can be highly produced.

Therefore, the method of producing an enzyme described in the eleventh aspect of the present invention is the same as the method of producing liquid koji described above.

The liquid koji obtained by the producing method of the present invention can be suitably used in producing fermented foods and drinks such as shochu. The liquid koji may be used instead of the solid koji, for instance, in the case of manufacturing sake, at the stage of preparing yeast or mash; in the case of manufacturing shochu, at the stage of preparing mash; in the case of manufacturing soy sauce, at the stage of piling; in the case of manufacturing miso, at the stage of preparing; in the case of manufacturing sweet sake, at the stage of preparing; and in the case of manufacturing amazake, at the stage of preparing.

In addition, a part of the resultant liquid koji can be used as starter for subsequent production of liquid koji. By producing liquid koji continuously in this manner, stable production can be achieved and production efficiency can be improved at the same time.

When fermented foods and drinks such as shochu are produced by using the above-mentioned liquid koji, all steps can be conducted in liquid phase. A method of producing fermented foods and drinks in liquid phase through the whole steps, for instance, when shochu is produced, is that corn, wheat, rice, potato, sugar cane and the like as raw material are heated at about 80°C to liquefy by dissolving with a heat-resistant enzyme preparation, the above liquid koji and yeast are added thereto to allow the mash to alcohol fermentation, and then it is distillated under normal pressure or reduced pressure and the like.

The liquid koji obtained by the method of the present invention has high enzymatic activity, so the liquid koji can be utilized for an enzyme preparation and a pharmaceutical such as a digestive agent. In this case, the resultant culture product of koji molds may be concentrated and purified to desired extent so as to form into a formulation with adding thereto an appropriate excipient, a thickening agent, a sweetener and the like.

In addition, by utilizing a promoter region of the gene of the amylolytic enzyme and the like of the koji molds, a desirable heteroprotein can be highly produced in culture product of the koji molds.

### EXAMPLES

Although the present invention will be more specifically described hereinafter with reference to Examples and the like, the present invention is not limited to these Examples and the like.

### Example 1

### Addition of inorganic nitrogen substance in production of liquid koji

The effect when potassium nitrate as inorganic nitrogen substance was added to the liquid medium was investigated as described below.

At first, three types of liquid media were prepared, in which crude barley was added respectively to water without addition (control) , to that with 0.2% (w/vol) potassiumnitrate, and to that with 0.4% (w/vol) potassium nitrate so that the amount of the crude barley was adjusted to 2% (w/vol).

Each 100 ml of the liquid media was placed in a 500-ml baffled conical flask and was autoclaved, and white koj i molds (Aspergillus kawachii IFO4308) cultured in advance in liquid medium was inoculated so that the amount thereof was adjusted to 1% (v/vol) for the liquid medium. As the crude barley, 95% polished Stirling barley made in Australia was used (this is basically true in the examples herein below).

Culture was then conducted for 48 hours at a temperature of 37 °C and a shaking speed of 100 rpm. After completion of the culture, each of the resulting culture products was measured for glucoamylase activity and acid-stable α-amylase activity. The glucoamylase and acid-stable α-amylase activities of the culture products obtained from culturing koji molds in the liquid media depending on the used amounts of potassium nitrate were shown in Table 1 and FIG. 1.

For measuring the enzymatic activity of glucoamylase, a saccharification power fractional quantification kit (manufactured by Kikkoman) was used. For measuring the enzymatic activity of acid-stable α-amylase, the method described in "Nagamine K. et al. : Biosci. Biotechnol. Biochem, 67, 2194-2202 (2003)" was slightly modified. That is, acid-unstable α-amylase was inactivated by treating the culture product with acid, and then acid-stable α-amylase activity was measured with the an α-amylase measurement kit (manufactured by Kikkoman). To be more specific, 9 ml of 100 mM acetic acid buffer (pH 3) was added to 1 ml of the culture solution, acid treatment was conducted at 37°C for 1 hour, and then measured with the α-amylase measurement kit (manufactured by Kikkoman).

As shown in Table 1 and FIG. 1, activities of the enzymes glucoamylase and acid-stable α-amylase were remarkably improved in both of the 0.2% added plot and 0.4% added plot where the liquid media were cultured with adding thereto the potassium nitrate as inorganic nitrogen substance, as compared to that in the control plot of without addition, and also the balance between the glucoamylase and the acid-stable α-amylase was good.

**[Table 1]**

| | Addition amount of KNO₃ (w/vol) | Enzymatic activity (U/ml) | |
|---|---|---|---|
| | | Glucoamylase (GA) | Acid-stable α-amylase (ASAA) |
| No. 1(Control) | Without addition | 32.6 | 2.5 |
| No. 2 | 0.20% | 124.3 | 8.7 |
| No.3 | 0.40% | 137.9 | 7.9 |

### Example 2

### Addition of a plurality of inorganic substances in production of liquid koji

The effect when a plurality of inorganic substances were added was investigated as described below.

Potassium nitrate or sodium nitrate as inorganic nitrogen substance, and potassium dihydrogen phosphate as inorganic salt were added to water in the compositions shown in Table 2. The addition amount of the sodium nitrate was calculated out from the molar concentration corresponding to 2.0% of potassium nitrate, that is, 20 mM, and was blended 1.7% so that the nitrate ion concentrations become the same. Water without inorganic nitrogen substance nor inorganic salt was used as the control.

To the raw material water prepared as described above was added crude barley as culture raw material in a concentration of 2% (w/vol), and 4 types of liquid media were prepared. Liquid culture of the white koj i molds was conducted under the same conditions as that in Example 1. After that, glucoamylase activity and acid-stable α-amylase activity were determined by the same method as that in Example 1. Table 2 and FIG. 2 show the results.

**[Table 2]**

| | Medium (2%-crude barley medium) | | | | Enzymatic activity (U/ml) | |
|---|---|---|---|---|---|---|
| | Inorganic nitrogen substance (w/vol) | | Inorganic salt (w/vol) | | Glucoamylase (GA) | Acid-stable α-amylase(ASAA) |
| No. 1 (Control) | - | | - | | 32.6 | 2.5 |
| No. 2 | 0.20% | KNO₃ | 0.27% | KH₂PO₄ | 143.6 | 8.8 |
| No. 3 | 0.17% | NaNO₃ | 0.27% | KH₂PO₄ | 125.6 | 7.7 |

As shown in Table 2 and FIG. 2, activities of the enzymes glucoamylase and acid-stable α-amylase were improved in the plots with adding thereto the inorganic nitrogen substance and inorganic salt as compared to the control plot of without addition.

### Example 3

### Addition of yeast cells or yeast autolysate in production of liquid koji

Liquid koj i was produced by using liquid medium to which yeast cells or yeast autolysate (i.e., yeast extract) was added.

### (1) Preparation of yeast cells or yeast autolysate to be added

Beer yeast recovered from a step of brewing beer was treated under the following conditions, to thereby obtain beer yeast cells and yeast autolysates (1) and (2) to be used in the production of liquid koji.

Yeast cells: The beer yeast cells obtained by dehydrating beer yeast to about a water content of 70% by means of centrifugation at 5,000 × g for 15 minutes.

Yeast autolysate (1): The yeast autolysate obtained by suspending beer yeast cells in an equal amount of water and treating the mixture at 52°C for 18 hours.

Yeast autolysate (2): The yeast autolysate obtained by suspending beer yeast cells in an equal amount of 1% lactic acid and treating the mixture at 52°C for 18 hours.

### (2) Preparation of liquid koji by using liquid medium to which yeast is added

Each of the yeast cells and yeast autolysates (1) and (2) which were prepared as described above was added to water so as to have concentrations of 0.20%, 0.50% and 1% (v/vol), respectively, to thereby prepare raw material water. Crude barley as culture raw material was added to each of the raw material water so as to have a concentration of 2% (w/vol), whereby preparing liquid medium. Liquid culture of the white koji molds was conducted under the same conditions as those in Example 1. After that, glucoamylase activity and acid-stable α-amylase activity were determined according to the same method as that in Example 1.

As the control, liquid medium (i.e., plot No. 1) was prepared by adding only crude barley to water in a concentration of only 2% (w/vol), and white koji molds were then inoculated thereto in the same manner as that in Example 1 to conduct liquid culture. Glucoamylase activity and acid-stable α-amylase activityof the resultant liquid koj i were determined in the same manner. Table 3 and FIG. 3 show the results.

**[Table 3]**

| | Medium (2%-crude barley medium) | | | Enzymatic activity (U/ml) | |
|---|---|---|---|---|---|
| | Yeast cells * | Yeast autolysate(1)* | Yeast autolysate2)* | Glucoamylase (GA) | Acid-stable α-amylase (ASAA) |
| NO. 1 | - | - | - | 16.3 | 1.0 |
| NO. 2 | 0.2% | - | - | 29.2 | 2.1 |
| NO. 3 | 0.5% | - | - | 48.2 | 2.1 |
| NO. 4 | 1.0% | - | - | 74.9 | 9.2 |
| NO. 5 | - | 0.2% | - | 35.3 | 2.5 |
| NO. 6 | - | 0.5% | - | 56.6 | 3.1 |
| NO. 7 | - | 1.0% | - | 110.0 | 9.6 |
| NO. 8 | - | - | 0.2% | 23.7 | 1.8 |
| NO. 9 | - | - | 0.5% | 59.1 | 5.2 |
| NO. 10 | - | - | 1.0% | 68.0 | 10.3 |

| | | | | | |
|---|---|---|---|---|---|
| *: unit is v/vol | | | | | |

### (3) Results

As shown in Table 3 and FIG. 3, both the glucoamylase activity and the acid-stable α-amylase activity were improved in all the experimental plots to which yeast cells themselves were added and in the experimental plots to which yeast autolysate was added, as compared to those in the control plot (i.e., plot No. 1) without addition. In particular, experimental plot No. 7 showed a good result. In addition, in every experimental plot, the glucoamylase activity and the acid-stable α-amylase activity were improved in proportion to the addition amount of the yeast cells or yeast autolysate.

### Example 4

### Addition in combination of inorganic nitrogen substance and/or inorganic salt with yeast cells

Potassium nitrate, potassium dihydrogen phosphate, and yeast cells were combined in the manner as shown in Table 4, and added to water to prepare raw material water. The used yeast cells were beer yeast cells themselves (i.e., the yeast cells prepared in Example 3) obtained by dehydrating the beer yeast recovered from a step of brewing beer to a water content of about 70% by means of centrifugation. As the control (i.e., plot No. 1), raw material water without addition was used.

Crude barley was added to the raw material water prepared with the combinations shown in Table 4 in a concentration of 2% (w/vol). White koji molds were inoculated to the liquid media in the same manner as that in Example 1 to conduct liquid culture. Glucoamylase activity and acid-stable α-amylase activity in those liquid media were determined, respectively. Table 4 and FIG. 4 show the results.

**[Table 4]**

| | Medium (2%-crude barley medium) | | | Enzymatic activity (U/ml) | |
|---|---|---|---|---|---|
| | KNO₃ (w/vol) | KH₂PO₄ (w/vol) | Yeast cells (v/vol) | Glucoamylase (GA) | Acid-stable α-amylase (ASAA) |
| NO. 1 (Control) | - | - | - | 32.6 | 0.8 |
| NO. 2 | 0.05% | - | - | 52.9 | 3.8 |
| NO. 3 | 0.10% | - | - | 91.7 | 4.6 |
| NO. 4 | 0.20% | - | - | 114.0 | 5.6 |
| NO. 5 | 0.40% | - | - | 137.9 | 6.9 |
| NO. 6 | - | - | 0.20% | 40.8 | 2.4 |
| NO. 7 | 0.05% | - | 0.20% | 60.3 | 3.7 |
| NO. 8 | 0.10% | - | 0.20% | 101.4 | 5.4 |
| NO. 9 | 0.20% | - | 0.20% | 103.6 | 6.4 |
| NO. 10 | 0.40% | - | 0.20% | 139.9 | 8.6 |
| NO. 11 | - | - | 0.50% | 44.5 | 3.7 |
| NO. 12 | 0.05% | - | 0.50% | 94.4 | 6.5 |
| NO. 13 | 0.10% | - | 0.50% | 94.9 | 6.9 |
| NO. 14 | 0.20% | - | 0.50% | 135.7 | 8.3 |
| NO. 15 | 0.40% | - | 0.50% | 154.0 | 10.4 |
| NO. 16 | 0.20% | 0.30% | - | 139.9 | 10.5 |
| NO. 17 | 0.20% | 0.30% | 0.20% | 160.7 | 11.6 |
| NO. 18 | 0.20% | 0.30% | 0.50% | 178.5 | 12.3 |

As shown in Table 4 and FIG. 4, both the glucoamylase activity and the acid-stable α-amylase activity were extremely high in all the experimental plots, as compared to those in the control plot (i.e., plot No. 1) without addition. In particular, in experimental plots No. 15 to No. 18, activities of the both enzymes were extremely high. From the results, it was thought that the combined use of the inorganic nitrogen substance and/or the inorganic salt with the yeast cells improved the nutrition balance in the liquid medium, so the filamentous fungi briskly produced the enzymes.

### Example 5

### Combination of barley bran, yeast cells, and inorganic substance

Barley bran and yeast cells, potassium nitrate and potassium dihydrogen phosphate were combined in the manner as shown in Table 5, and added to water to prepare raw material water for liquid medium. The used barley bran was the one recovered from a pearling step for 70% polished barley (Stirling, made in Australia), and contained barley husks and bran. The used yeast cells were beer yeast cells themselves (i.e., the yeast cells prepared in Example 3) obtained by dehydrating beer yeast recovered from a step of brewing beer to a water content of about 70% by means of centrifugation. As the control, raw material water without addition was used.

Crude barley was added to the raw material water prepared with the combinations as shown in Table 5 in a concentration of 2% (w/vol) . White koji molds were inoculated to the liquid media in the same manner as that in Example 1 to conduct liquid culture. Glucoamylase activity and acid-stable α-amylase activity in those liquid media were determined, respectively, in the same manner. Table 5 and FIG. 5 show the results.

**[Table 5]**

| | Medium (2%-crude barley medium) | | | Enzymatic activity (U/ml) | |
|---|---|---|---|---|---|
| | Inorganic compound | Barley bran (w/vol) | Yeast cells (v/vol) | Glucoamylase (GA) | Acid-stable α-amylase (ASAA) |
| NO. 1 (Control) | - | - | - | 25.7 | 0.9 |
| NO. 2 | - | 0.1% | 0.1% | 43.5 | 2.9 |
| NO. 3 | - | 0.5% | 0.5% | 54.6 | 3.9 |
| NO. 4 | - | 1.0% | 1.0% | 111.0 | 13.6 |
| NO. 5 | - | 2.0% | 2.0% | 50.9 | 8.5 |
| NO. 6 | - | 1.0% | - | 77.6 | 8.9 |
| NO. 7 | - | - | 1.0 | 92.3 | 9.5 |
| NO. 8 | ○ | 1.0% | - | 106.0 | 10.3 |
| NO. 9 | ○ | - | 1.0% | 318.6 | 15.5 |

| | | | | | |
|---|---|---|---|---|---|
| ○: an experimental plot to which 0.2% (w/vol) of potassium nitrate and 0.3% (w/vol) of potassium dihydrogen phosphate were added. | | | | | |

As shown in Table 5 and FIG. 5, both the glucoamylase activity and the acid-stable α-amylase activity were extremely high in all the experimental plots, as compared to those in the control plot (i.e., plot No. 1) without addition. In particular, in experimental plot No. 4, the activities of both enzymes were extremely high, and the balance was good. From the results, it was thought that the combined use of the barley bran with the yeast cells improved the nutrition balance in the liquid medium, so the filamentous fungi briskly produced the enzymes.

It should be noted that the combined use of the barley bran or yeast cells with the inorganic nitrogen substance and inorganic salt (i.e., plots No. 8 and No. 9) showed good result.

### Example 6

### Addition in combination of barley husks and yeast cells

Potassium nitrate, potassium dihydrogen phosphate, barley husks, and yeast cells were combined in a manner as shown in Table 6, and added to raw water to prepare raw material water for liquid medium. The used barley husks were those obtained by sifting barley bran obtained from a pearling step for 70% polished barley with a 2 mm-meshed sieve, and then recovering only barley husks. In addition, the used compressed yeast was beer yeast cells themselves (i.e., the yeast cells prepared in Example 3) obtained by dehydrating the beer yeast recovered from a step of brewing beer to a water content of about 70% by means of centrifugation. As the control, raw water without addition (i.e., plot No. 1) was used.

Crude barley was added to the raw material water prepared with the combinations as shown in Table 6 in a concentration of 2% (w/vol) . White koji molds were inoculated to the liquid media in the same manner as that in Example 1 to conduct liquid culture. Glucoamylase activity and acid-stable α-amylase activity in those liquid media were determined, respectively. Table 6 and FIG. 6 show the results.

**[Table 6]**

| | Medium | | Enzymatic activity (U/ml) | |
|---|---|---|---|---|
| | Yeast cells (v/vol) | Barley husks (w/vol) | Glucoamylase (GA) | Acid-stable α-amylase (ASAA) |
| NO. 1 (Control) | - | - | 33.9 | 0.8 |
| NO. 2 | - | 0.1% | 65.3 | 5.5 |
| NO. 3 | - | 0.5% | 55.2 | 6.3 |
| NO. 4 | - | 1.0% | 48.5 | 6.9 |
| NO. 5 | - | 2.0% | 44.3 | 4.6 |
| NO. 6 | 1.0% | 0.1% | 94.4 | 9.9 |
| NO. 7 | 1.0% | 0.5% | 79.8 | 9.4 |
| NO. 8 | 1.0% | 1.0% | 85.8 | 11.0 |
| NO. 9 | 1.0% | 2.0% | 82.3 | 7.1 |

As shown in Table 6 and FIG. 6, both the glucoamylase activity and the acid-stable α-amylase activity were extremely high in all the experimental plots, as compared to those in the control plot (i.e., plot No. 1) without addition. In particular, in experimental plots No. 6 to No. 9, the activities of the both enzymes were extremely high, and the balance was good. From the results, it was thought that the combined use of the barley husks with the yeast cells improved the nutrition balance in the liquid medium, so the filamentous fungi briskly produced the enzymes.

### Example 7

### Effect of adding sulfate salt in producing liquid koji

Liquid koji was produced by the method described below, and enzymatic activities thereof were determined.

### 1. Method of preculture

8 g of 65% polished barley (Stirling, made in Australia) and 100 ml of water were filled into a 500-ml baffled conical flask and autoclaved at 121°C for 15 minutes. After being cooled, white koji molds (*Aspergillus kawachii* NBRC4308) was inoculated at 1 x 10⁶/ml into the preculture medium and cultured by shaking at 37 °C and 100 rpm for 24 hours. The medium was defined as preculture medium.

### 2. Method of main culture

100 ml of liquid medium was prepared respectively as five experimental plots, each containing 2.0% (w/vol) of 98% polished barley (crude barley, Stirling, made in Australia), 0.2% (w/vol) of potassium nitrate, 0.3% (w/vol) of potassium dihydrogen phosphate, 0.1% (w/vol) of magnesium sulfate heptahydrate, and 0.082% (w/vol) of magnesium chloride hexahydrate at the composition ratio as shown in Table 7 . These five liquid media were respectively filled into a 500 ml baffled conical flask and autoclaved at 121°C for 15 minutes to sterilize.

After being cooled, 1 ml of the preculture liquid was inoculated to the main culture medium, and the whole was cultured by shaking at 37°C and 100 rpm for 48 hours. Note that the addition amount of magnesium chloride hexahydrate was calculated from the molar concentration corresponding to 0.1% of magnesium sulfate heptahydrate, that is, 8.12 mM, so that the magnesium concentrations of media in each experimental plot became equal.

**[Table 7]**

| | Medium composition | | | | |
|---|---|---|---|---|---|
| Experimental plot 1 | 2%-crude barley | 0.2%KNO₃ | 0.3%KH₂PO₄ | - | - |
| Experimental plot 2 | 2%-crude barley | 0.2%KNO₃ | 0.3%KH₂PO₄ | 0.1%MgSO₄· 7H₂O | - |
| Experimental plot 3 | 2%-crude barley | 0.2%KNO₃ | 0.3%KH₂PO₄ | - | 0.082%MgCl₂· 6H₂O |
| Experimental plot 4 | 2%-crude barley | - | 0.3%KH₂PO₄ | 0.1%MgSO₄· 7H₂O | - |
| Experimental plot 5 | 2%-crude barley | 0.2%KNO₃ | - | 0.1%MgSO₄· 7H₂O | - |

### 3. Method of determining enzymatic activity

After completion of culture, activities of glucoamylase (GA) and acid-stable α-amylase (ASAA) which were amylolytic enzymes were determined.

The glucoamylase (GA) activity was determined by using a saccharification power fractional quantification kit (manufactured by Kikkoman).

For determining the acid-stable α-amylase (ASAA) activity, the method described in Sudo S. et al: J. Ferment. Bioeng., 76, 105-110 (1993), Sudo S. et al: J. Ferment. Bioeng., 77, 483-489 (1994), and Shigetoshi Sudo et al: Journal of the Brewing Society of Japan, 89, 768-774(1994) was slightly modified. That is, acid-unstable α-amylase activity was inactivated by treating the culture product with acid, and then acid-stable α-amylase activity was measured with the α-amylase measurement kit (manufactured by Kikkoman). To be more specific, 9 ml of a 100 mM acetic acid buffer solution (pH 3) was added to 1 ml of culture solution, and acid treatment was conducted at 37 °C for 1 hour, and then measured with the α-amylase measurement kit (manufactured by Kikkoman).

At the same time, activity of cellulase (CEL) that is a cellulolytic enzyme and activity of acid carboxypeptidase (ACP) that is one of proteolytic enzymes were determined.

The cellulase (CEL) activity was measured by the method of quantitating an amount of the reduced saccharide, which is generated by hydrolysing carboxymethyl cellulose (CMC) as a substrate, with the dinitrosalicylic acid (DNS) method. To be more specific, 1 ml of the culture solution was added to 1 ml of 1% CMC substrate solution (low viscosity^{™} produced by Sigma-Aldrich was dissolved in a 100 mM acetic acid buffer solution (pH 5)), and the whole was subjected to enzymatic reaction at 40°C precisely for 10 minutes. After that, to the mixture was added 4 ml of the DNS reagent containing 0.75% dinitrosalicylic acid, 1.2% sodium hydroxide, 22.5% potassium sodium tartrate tetrahydrate and 0.3% lactose monohydrate, and the whole was well mixed, to thereby terminate the reaction. In order to quantitate the amount of reduced saccharide in the reaction terminated solution, the reaction terminated solution was heated in a boiling water bath precisely for 15 minutes. Subsequently, after the solution was cooled to room temperature, absorbance at 540 nm was determined, to thereby quantitate the amount of the reduced saccharide corresponding to glucose. One unit of cellulase (CEL) activity was represented by the amount of enzyme required for producing reduced saccharide corresponding to 1 µmol of glucose per minute.

The acid carboxypeptidase (ACP) activity was determined by using an acid carboxypeptidase measurement kit (manufactured by Kikkoman).

FIG. 7 shows the determination results.

### 4. Results

As shown in FIG. 7A, glucoamylase activity was significantly improved in experimental plot 2 that is the plot to which magnesium sulfate was added. In addition, as shown in FIGS. 7C and 7D, activities of cellulase and acid carboxypeptidase were also improved in experimental plot 2 that is the plot to which magnesium sulfate was added. On the other hand, the activity was not improved in experimental plot 3 to which magnesium chloride, that is a kind of the same magnesium salts was added, so it was suggested that a sulfate radical serves as amain reason for these advantageous effects in enzyme productivity.

In addition, the advantageous effects in enzyme productivity was not observed in experimental plots 4 and 5 to which magnesium sulfate was added but no potassium nitrate nor potassium dihydrogen phosphate. Accordingly, it was found that enzyme productivity was remarkably improved when nitrate salts, phosphate salts and sulfate salts were incorporated at the same time.

As described above, when koj i molds are cultured by using liquid medium to which are added the cereals the surface of which is covered with husks (e.g., crude barley), nitrate salts, phosphate salts and sulfate salts, there can be produced liquid koji containing with high yield cellulase that is a cellulolytic enzyme and acid carboxypeptidase that is a proteolytic enzyme in addition to enzymes required for producing shochu and the like such as glucoamylase and acid-stable α-amylase.

Owing to the high yield of the cellulolytic enzymes, decrease in mash viscosity or increase in alcohol yield during the production of shochu can be expected, and if amino acid components in the shochu mash is increased owing to the high yield of the proteolytic enzymes, shochu with gorgeous flavor can be produced.

In addition, according to the method of the present invention, it is expected that enzymes produced by koj i molds, such as the amylolytic enzymes, cellulolytic enzymes and proteolytic enzymes other than those determined herein, are generally produced with high yield.

### Industrial applicability

According to the present invention, it is possible that not only productivity of amylolytic enzymes in liquid koji is significantly improved, but liquid koji containing with high yield cellulolytic enzymes and proteolytic enzymes, is produced. Further, liquid culture can strictly control as compared to solid culture, so liquid koji having quality stability can be efficiently produced at low cost.

By using the liquid koji produced according to the present invention for producing fermented foods and drinks such as shochu, alcohol yields and amino acid production amounts can be increased, whereby fermented foods and drinks each having gorgeous flavor can efficiently be produced.

Moreover, the cereals used in the present invention are unpolished or polished to an extent in which at least husks remain on the surface. Thus, improvement in raw material availability and in yield can be expected.

## Claims

1. A method of producing liquid koji having enhanced activity of enzymes, comprising culturing white koji molds and/or black koji molds in a liquid medium which contains (i) a cereal the surface of which is entirely covered with husks and (ii) at least one nitrogen source selected from the group consisting of potassium nitrate, sodium nitrate, yeast cells, treated products of yeast cells, cereal husks and cereal bran.

2. The method of producing liquid koj i having enhanced activity of enzymes according to claim 1, wherein the enzymes comprise glucoamylase and acid-stable α-amylase.

3. The method of producing liquid koj i having enhanced activity of enzymes according to claim 1, wherein the liquid medium contains:
a cereal the surface of which is entirely covered with husks;
at least one nitrogen source selected from the group consisting of potassium nitrate and sodium nitrate;
at least one phosphate salt selected from the group consisting of potassium dihydrogen phosphate and ammonium phosphate; and
at least one sulfate salt selected from the group consisting of magnesium sulfate heptahydrate, iron sulfate heptahydrate and ammonium sulfate.

4. The method of producing liquid koj i having enhanced activity of enzymes according to claim 3, wherein the liquid medium contains the at least one nitrogen source in a concentration of 0.1 to 2.0% (w/vol).

5. The method of producing liquid koji having enhanced activity of enzymes according to claim 3, wherein the liquid medium contains the at least one phosphate salt in a concentration of 0.05 to 1.0% (w/vol).

6. The method of producing liquid koj i having enhanced activity of enzymes according to claim 3, wherein the liquid medium contains the at least one sulfate salt in a concentration of 0.01 to 0.5% (w/vol).

7. The method of producing liquid koj i having enhanced activity of enzymes according to claim 1, wherein the cereal comprises at least one selected from the group consisting of crude rice, rice with all chaffs, rice with part of chaffs and barley having a polishing ratio of not less than 93%.

8. The method of producing liquid koj i having enhanced activity of enzymes according to claim 1, wherein the white koji molds comprise *Aspergillus kawachii*.

9. The method of producing liquid koj i having enhanced activity of enzymes according to claim 1, wherein the black koji molds comprise at least one selected from the group consisting of *Aspergillus awamori* and *Aspergillus niger*.

10. The method of producing liquid koj i having enhanced activity of enzymes according to claim 1, wherein the cereal comprises barley.

11. A method of producing at least one enzyme selected from the group consisting of glucoamylase and acid-stable α-amylase, comprising producing the at least one enzyme by culturing white koj i molds and/or black koj i molds in a liquid medium which contains (i) a cereal the surface of which is entirely covered with husks and (ii) at least one nitrogen source selected from the group consisting of potassium nitrate, sodium nitrate, yeast cells, treated products of yeast cells, cereal husks and cereal bran.

12. A method of producing at least one enzyme selected from the group consisting of glucoamylase, acid-stable α-amylase, cellulase and acidic carboxypeptidase comprising producing the at least one enzyme by culturing white koj i molds and/or black koji molds in a liquid medium which contains:
a cereal the surface of which is entirely covered with husks;
at least one nitrogen source selected from the group consisting of potassium nitrate and sodium nitrate;
at least one phosphate salt selected from the group consisting of potassium dihydrogen phosphate and ammonium phosphate; and
at least one sulfate salt selected from the group consisting of magnesium sulfate heptahydrate, iron sulfate heptahydrate and ammonium sulfate.

13. The method of producing enzymes according to claim 12, wherein the liquid medium contains the at least one nitrogen source in a concentration of 0.1 to 2.0% (w/vol).

14. The method of producing enzymes according to claim 12, wherein the liquid medium contains the at least one phosphate salt in a concentration of 0.05 to 1.0% (w/vol).

15. The method of producing enzymes according to claim 12, wherein the liquid medium contains the at least one sulfate salt in a concentration of 0.01 to 0.5% (w/vol).

16. The method of producing enzymes according to claim 11 or 12, wherein the cereal comprises at least one selected from the group consisting of crude rice, rice with all chaffs, rice with part of chaffs and barley having a polishing ratio of not less than 93%.

17. The method of producing enzymes according to claim 11 or 12, wherein the white koji molds comprise *Aspergillus kawachii.*

18. The method of producing enzymes according to claim 11 or 12, wherein the black koji molds comprise at least one selected from the group consisting of *Aspergillus awamori* and *Aspergillus niger.*

19. The method of producing enzymes according to claim 11 or 12, wherein the cereal comprises barley.

## Patentansprüche

1. Verfahren zum Produzieren von flüssigem Koji mit erhöhter Aktivität von Enzymen, umfassend ein Kultivieren von weißen Koji-Schimmelpilzen und/oder schwarzen Koji-Schimmelpilzen in einem flüssigen Medium, das
(i) ein Getreide, dessen Oberfläche vollständig mit Hülsen bedeckt ist, und
(ii) mindestens eine Stickstoffquelle, ausgewählt aus der Gruppe, bestehend aus Kaliumnitrat, Natriumnitrat, Hefezellen, behandelten Produkten von Hefezellen, Getreidehülsen und Getreidekleie, enthält.

2. Verfahren zum Produzieren von flüssigem Koji mit erhöhter Aktivität von Enzymen gemäß Anspruch 1, wobei die Enzyme Glucoamylase und säurestabile α-Amylase umfassen.

3. Verfahren zum Produzieren von flüssigem Koji mit erhöhter Aktivität von Enzymen gemäß Anspruch 1, wobei das flüssige Medium:
ein Getreide, dessen Oberfläche vollständig mit Hülsen bedeckt ist,
mindestens eine Stickstoffquelle, ausgewählt aus der Gruppe, bestehend aus Kaliumnitrat und Natriumnitrat,
mindestens ein Phosphatsalz, ausgewählt aus der Gruppe, bestehend aus Kaliumdihydrogenphosphat und Ammoniumphosphat, und
mindestens ein Sulfatsalz, ausgewählt aus der Gruppe, bestehend aus Magnesiumsulfat-Heptahydrat, Eisensulfat-Heptahydrat und Ammoniumsulfat,
enthält.

4. Verfahren zum Produzieren von flüssigem Koji mit erhöhter Aktivität von Enzymen gemäß Anspruch 3, wobei das flüssige Medium die mindestens eine Stickstoffquelle in einer Konzentration von 0,1 bis 2,0% (Gew./Vol.) enthält.

5. Verfahren zum Produzieren von flüssigem Koji mit erhöhter Aktivität von Enzymen gemäß Anspruch 3, wobei das flüssige Medium das mindestens eine Phosphatsalz in einer Konzentration von 0,05 bis 1,0% (Gew./Vol.) enthält.

6. Verfahren zum Produzieren von flüssigem Koji mit erhöhter Aktivität von Enzymen gemäß Anspruch 3, wobei das flüssige Medium das mindestens eine Sulfatsalz in einer Konzentration von 0,01 bis 0,5% (Gew./Vol.) enthält.

7. Verfahren zum Produzieren von flüssigem Koji mit erhöhter Aktivität von Enzymen gemäß Anspruch 1, wobei das Getreide mindestens eines, ausgewählt aus der Gruppe, bestehend aus unbearbeitetem Reis, Reis mit der gesamten Spreu, Reis mit einem Teil der Spreu und Gerste mit einem Polierverhältnis von nicht weniger als 93%, umfasst.

8. Verfahren zum Produzieren von flüssigem Koji mit erhöhter Aktivität von Enzymen gemäß Anspruch 1, wobei die weißen Koji-Schimmelpilze *Aspergillus kawachii* umfassen.

9. Verfahren zum Produzieren von flüssigem Koji mit erhöhter Aktivität von Enzymen gemäß Anspruch 1, wobei die schwarzen Koji-Schimmelpilze mindestens einen, ausgewählt aus der Gruppe, bestehend aus *Aspergillus awamori* und *Aspergillus niger,* umfassen.

10. Verfahren zum Produzieren von flüssigem Koji mit erhöhter Aktivität von Enzymen gemäß Anspruch 1, wobei das Getreide Gerste umfasst.

11. Verfahren zum Produzieren mindestens eines Enzyms, ausgewählt aus der Gruppe, bestehend aus Glucoamylase und säurestabiler α-Amylase, wobei das Verfahren ein Produzieren des mindestens einen Enzyms durch Kultivieren von weißen Koji-Schimmelpilzen und/oder schwarzen Koji-Schimmelpilzen in einem flüssigen Medium umfasst, das (i) ein Getreide, dessen Oberfläche vollständig mit Hülsen bedeckt ist, und (ii) mindestens eine Stickstoffquelle, ausgewählt aus der Gruppe, bestehend aus Kaliumnitrat, Natriumnitrat, Hefezellen, behandelten Produkten von Hefezellen, Getreidehülsen und Getreidekleie, enthält.

12. Verfahren zum Produzieren mindestens eines Enzyms, ausgewählt aus der Gruppe, bestehend aus Glucoamylase, säurestabiler α-Amylase, Cellulase und saurer Carboxypeptidase, wobei das Verfahren ein Produzieren des mindestens einen Enzyms durch Kultivieren von weißen Koji-Schimmelpilzen und/oder schwarzen Koji-Schimmelpilzen in einem flüssigen Medium umfasst, das:
ein Getreide, dessen Oberfläche vollständig mit Hülsen bedeckt ist,
mindestens eine Stickstoffquelle, ausgewählt aus der Gruppe, bestehend aus Kaliumnitrat und Natriumnitrat,
mindestens ein Phosphatsalz, ausgewählt aus der Gruppe, bestehend aus Kaliumdihydrogenphosphat und Ammoniumphosphat, und
mindestens ein Sulfatsalz, ausgewählt aus der Gruppe, bestehend aus Magnesiumsulfat-Heptahydrat, Eisensulfat-Heptahydrat und Ammoniumsulfat,
enthält.

13. Verfahren zum Produzieren von Enzymen gemäß Anspruch 12, wobei das flüssige Medium die mindestens eine Stickstoffquelle in einer Konzentration von 0,1 bis 2,0% (Gew./Vol.) enthält.

14. Verfahren zum Produzieren von Enzymen gemäß Anspruch 12, wobei das flüssige Medium das mindestens eine Phosphatsalz in einer Konzentration von 0,05 bis 1,0% (Gew./Vol.) enthält.

15. Verfahren zum Produzieren von Enzymen gemäß Anspruch 12, wobei das flüssige Medium das mindestens eine Sulfatsalz in einer Konzentration von 0,01 bis 0,5% (Gew./Vol.) enthält.

16. Verfahren zum Produzieren von Enzymen gemäß Anspruch 11 oder 12, wobei das Getreide mindestens eines, ausgewählt aus der Gruppe, bestehend aus unbearbeitetem Reis, Reis mit der gesamten Spreu, Reis mit einem Teil der Spreu und Gerste mit einem Polierverhältnis von nicht weniger als 93%, umfasst.

17. Verfahren zum Produzieren von Enzymen gemäß Anspruch 11 oder 12, wobei die weißen Koji-Schimmelpilze *Aspergillus kawachii* umfassen.

18. Verfahren zum Produzieren von Enzymen gemäß Anspruch 11 oder 12, wobei die schwarzen Koji-Schimmelpilze mindestens einen, ausgewählt aus der Gruppe, bestehend aus *Aspergillus awamori* und *Aspergillus niger,* umfassen.

19. Verfahren zum Produzieren von Enzymen gemäß Anspruch 11 oder 12, wobei das Getreide Gerste umfasst.

## Revendications

1. Procédé pour la production de koji liquide ayant une activité d'enzymes renforcée, comprenant la culture de moisissures de koji blanc et/ou de moisissures de koji noir dans un milieu liquide qui contient (i) une céréale dont la surface est entièrement couverte avec des cosses et (ii) au moins une source d'azote choisie dans le groupe consistant en nitrate de potassium, nitrate de sodium, cellules de levure, produits du traitement de cellules de levure, cosses de céréales et son de céréales.

2. Procédé pour la production de koji liquide ayant une activité d'enzymes renforcée selon la revendication 1, dans lequel les enzymes comprennent la glucoamylase et l'α-amylase stable en milieu acide.

3. Procédé pour la production de koji liquide ayant une activité d'enzymes renforcée selon la revendication 1, dans lequel le milieu liquide contient:
une céréale dont la surface est entièrement couverte avec des cosses;
au moins une source d'azote choisie dans le groupe consistant en du nitrate de potassium et du nitrate de sodium;
au moins un sel de phosphate choisi dans le groupe consistant en phosphate monopotassique et phosphate d'ammonium; et
au moins un sel de sulfate choisi dans le groupe consistant en sulfate de magnésium heptahydraté, sulfate de fer heptahydraté et sulfate d'ammonium.

4. Procédé pour la production de koji liquide ayant une activité d'enzymes renforcée selon la revendication 3, dans lequel le milieu liquide contient la au moins une source d'azote dans une concentration de 0,1 à 2,0% (en poids/volume).

5. Procédé pour la production de koji liquide ayant une activité d'enzymes renforcée selon la revendication 3, dans lequel le milieu liquide contient le au moins un sel de phosphate dans une concentration de 0,05 à 1,0% (en poids/volume).

6. Procédé pour la production de koji liquide ayant une activité d'enzymes renforcée selon la revendication 3, dans lequel le milieu liquide contient le au moins un sel de sulfate dans une concentration de 0,01 à 0,5% (en poids/volume).

7. Procédé pour la production de koji liquide ayant une activité d'enzymes renforcée selon la revendication 1, dans lequel la céréale comprend au moins une céréale choisie dans le groupe consistant en riz brut, riz avec toutes ses glumes, riz avec une partie des glumes et orge ayant un rapport de polissage d'au moins 93%.

8. Procédé pour la production de koji liquide ayant une activité d'enzymes renforcée selon la revendication 1, dans lequel les moisissures de koji blanc comprennent de l'*Aspergillus kawachii.*

9. Procédé pour la production de koji liquide ayant une activité d'enzymes renforcée selon la revendication 1, dans lequel les moisissures de koji noir comprennent au moins une moisissure choisie dans le groupe consistant en *Aspergillus awamori* et *Aspergillus niger.*

10. Procédé pour la production de koji liquide ayant une activité d'enzymes renforcée selon la revendication 1, dans lequel la céréale comprend de l'orge.

11. Procédé pour la production d'au moins une enzyme choisie dans le groupe consistant en glucoamylase et α-amylase stable en milieu acide, comprenant la production de la au moins une enzyme par culture de moisissures de koji blanc et/ou de moisissures de koji noir dans un milieu liquide qui contient (i) une céréale dont la surface est entièrement couverte avec des cosses et (ii) au moins une source d'azote choisie dans le groupe consistant en nitrate de potassium, nitrate de sodium, cellules de levure, produits du traitement de cellules de levure, cosses de céréales et son de céréales.

12. Procédé pour la production d'au moins une enzyme choisie dans le groupe consistant en glucoamylase, α-amylase stable en milieu acide, cellulase et carboxypeptidase acide comprenant la production de la au moins une enzyme par culture de moisissures de koji blanc et/ou de moisissures de koji noir dans un milieu liquide qui contient:
une céréale dont la surface est entièrement couverte avec des cosses;
au moins une source d'azote choisie dans le groupe consistant en du nitrate de potassium et du nitrate de sodium;
au moins un sel de phosphate choisi dans le groupe consistant en phosphate monopotassique et phosphate d'ammonium; et
au moins un sel de sulfate choisi dans le groupe consistant en sulfate de magnésium heptahydraté, sulfate de fer heptahydraté et sulfate d'ammonium.

13. Procédé pour la production d'enzymes selon la revendication 12, dans lequel le milieu liquide contient la au moins une source d'azote dans une concentration de 0,1 à 2,0% (en poids/volume).

14. Procédé pour la production d'enzymes selon la revendication 12, dans lequel le milieu liquide contient le au moins un sel de phosphate dans une concentration de 0,05 à 1,0% (en poids/volume).

15. Procédé pour la production d'enzymes selon la revendication 12, dans lequel le milieu liquide contient le au moins un sel de sulfate dans une concentration de 0,01 à 0,5% (en poids/volume).

16. Procédé pour la production d'enzymes selon la revendication 11 ou 12, dans lequel la céréale comprend au moins une céréale choisie dans le groupe consistant en riz brut, riz avec toutes ses glumes, riz avec une partie des glumes et orge ayant un rapport de polissage d'au moins 93%.

17. Procédé pour la production d'enzymes selon la revendication 11 ou 12, dans lequel les moisissures de koji blanc comprennent de *l'Aspergillus kawachii.*

18. Procédé pour la production d'enzymes selon la revendication 11 ou 12, dans lequel les moisissures de koji noir comprennent au moins une moisissure choisie dans le groupe consistant en *Aspergillus awamori* et *Aspergillus niger.*

19. Procédé pour la production d'enzymes selon la revendication 11 ou 12, dans lequel la céréale comprend de l'orge.
